# EUROPEAN PATENT SPECIFICATION

(11) **EP 1 414 364 B1**
(45) Date of publication and mention of the grant of the patent: **10.09.2008**
(21) Application number: 02761962.6
(22) Date of filing: 16.04.2002
(51) Int. Cl.: A61C 5/08, A61C 5/10, A61C 13/087, A61C 13/20

(54) **ACETAL RESIN CROWNS FOR CHILDREN**
ACETAL-HARZ-KRONEN FÜR KINDER
COURONNES EN POLYACETAL POUR ENFANTS

(30) Priority: 17.04.2001 IL 14265701; 11.07.2001 US 903096
(43) Date of publication of application: 06.05.2004
(73) Proprietor: Uri-Dent Ltd., Ashkelon 78172 (IL)
(72) Inventor: ZILBERMAN, Uri L., Nes Ziona 74019 (IL)
(74) Representative: Portal, Gérard
(86) International application number: PCT/IL2002/000310
(87) International publication number: WO 2002/083022

(56) References cited:
- EP-A- 0 030 850
- GB-A- 639 700
- US-A- 5 346 397
- US-A- 5 672 305
- US-A- 5 730 926
- US-B1- 6 186 790
- PATENT ABSTRACTS OF JAPAN vol. 1999, no. 13, 30 November 1999 (1999-11-30) -& JP 11 216148 A (SHIYUUKAI), 10 August 1999 (1999-08-10)

## Description

### FIELD OF THE INVENTION

The present invention relates to tooth prostheses generally and more particularly to crowns.

### BACKGROUND OF THE INVENTION

The following U. S. Patents and publications are believed to represent the current state of the art: 4,129,946; 5,552,390; 5,487,663; 5,624,261; 5,709,548; 8,106, 295;

JP 11 218148 discloses a dental crown produced with parallel sides without any sign of undercut and without any indication of flexibility, the side surfaces being directed outwardly. The top surface is made of at least one further resin precoated body 31, 32, see figures 5-7.

US-A-5,346,397 and EP-A-0 030 850 relate to porcelain or ceramic dental crowns which are not flexible.

### SUMMARY OF THE INVENTION

The present invention seeks to provide a mass-produced, tooth colored prefabricated crown, particularly useful in pediatric dentistry for treatment of primary teeth and permanent molars having extensive carious lesions, as defined in claims 1 and 2.

There is thus provided in accordance with the present Invention an injection molded dental crown formed of an acetal homopolymer, which preferably includes Polioxymethylene (POM) Thermoplastic Homopolymer.

in accordance with the present invention, the injection molded dental crown is formed with flexible depending side surfaces at least one of which defines an undercut.

The present invention crown can be produced according to a method for mass producing dental crowns comprising: providing a multi-element mold, employing the multi-element mold to injection mold the crown including depending side surfaces, at least one of which defines an undercut.
In accordance with a preferred embodiment not being part of the present invention, the multi-element mold includes an ejector which is operative to eject the molded crown following opening of the multi-element mold.

### BRIEF DESCRIPTION OF THE DRAWINGS

The present invention will be understood and appreciated more fully from the following detailed description, taken in conjunction with the drawings in which :
Fig. 1 is a simplified pictorial illustration of a dental crown formed of accetal homopolymer;
Fig. 2 is a sectional illustration of the dental crown of Fig. 1, taken along lines II - II in Fig. 1; and
Figs. 3A, 3B and 3C are each simplified pictorial illustrations of an apparatus for manufacturing a dental crown from acetal homopolymer resin in three operative orientations.

### DETAILED DESCRIPTION OF A PREFERRED EMBODIMENT

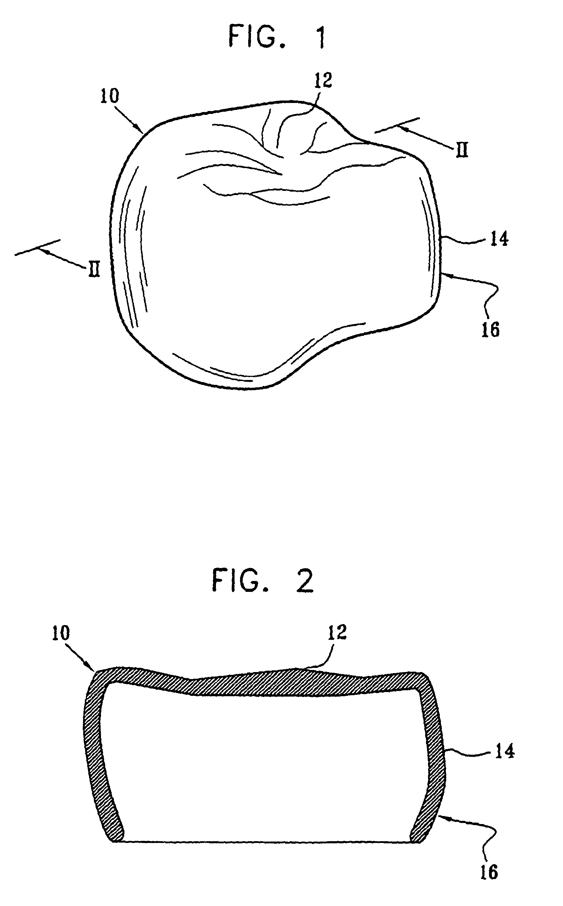

Reference is now made to Fig. 1, which is a simplified pictorial illustration of a dental crown formed of acetal homopolymer resin and to Fig. 2, which is a sectional illustration of the dental crown of Fig. 1, taken along lines II - II in Fig. 1.

As seen in Figs. 1 and 2, there is provided in accordance with the present invention an injection molded dental crown 10 formed of an acetal homopolymer resin. The material for the crown is acetal homopolymer resin (DELRIN^{®}) which is commercially available from DuPont.

As can be readily seen in Figs. 1 and 2, the dental crown 10 is formed with a generally conventionally tooth shaped top surface 12 and flexible depending side surfaces 14 at least one of which defines an undercut 16. Crown 10 may readily be mounted, by conventional methods, such as through the use of dental cement in the mouth of a patient, typically a child, as part of treatment of primary teeth and permanent molars having extensive carious lesions. It is a preferred feature that crown 10 is of a color which generally matches the patient's teeth.

The crown of the present invention is characterized by high tensile strength, high impact resistance and stiffness, excellent fatigue endurance and resistance to moisture, excellent dimensional stability and sufficient resilience and resistance to creep. It has the natural appearance of a vital tooth

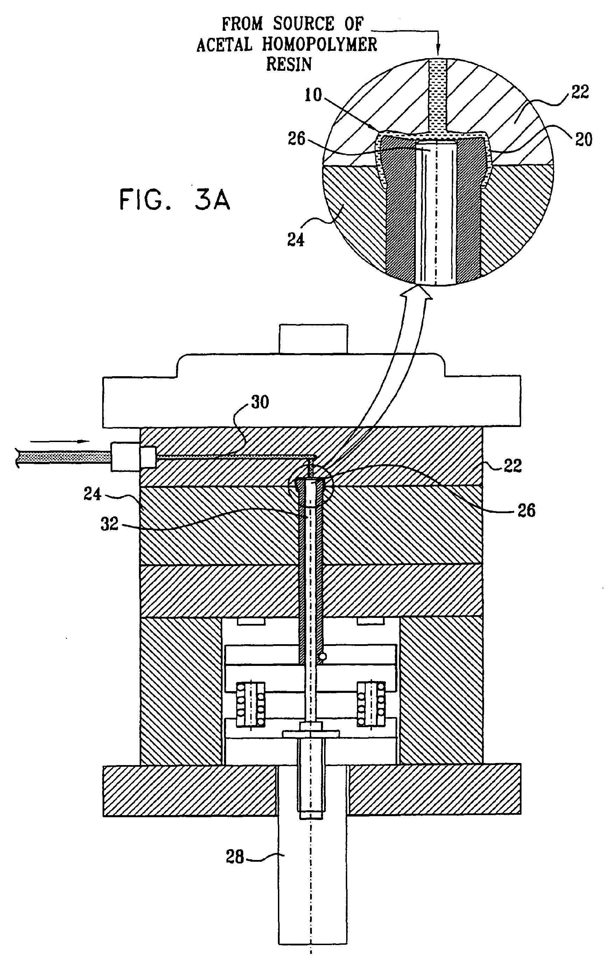

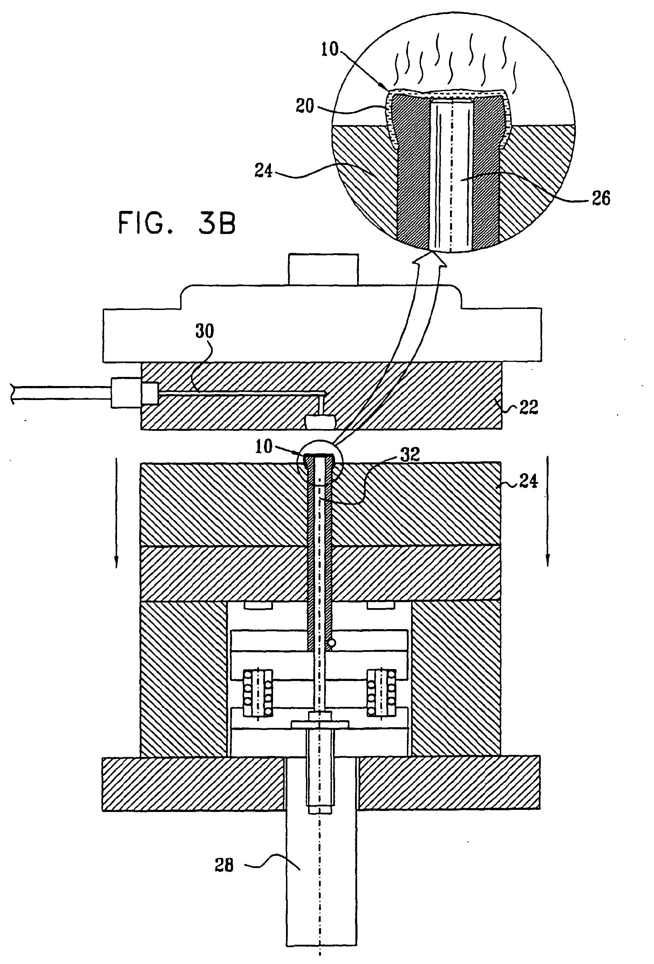

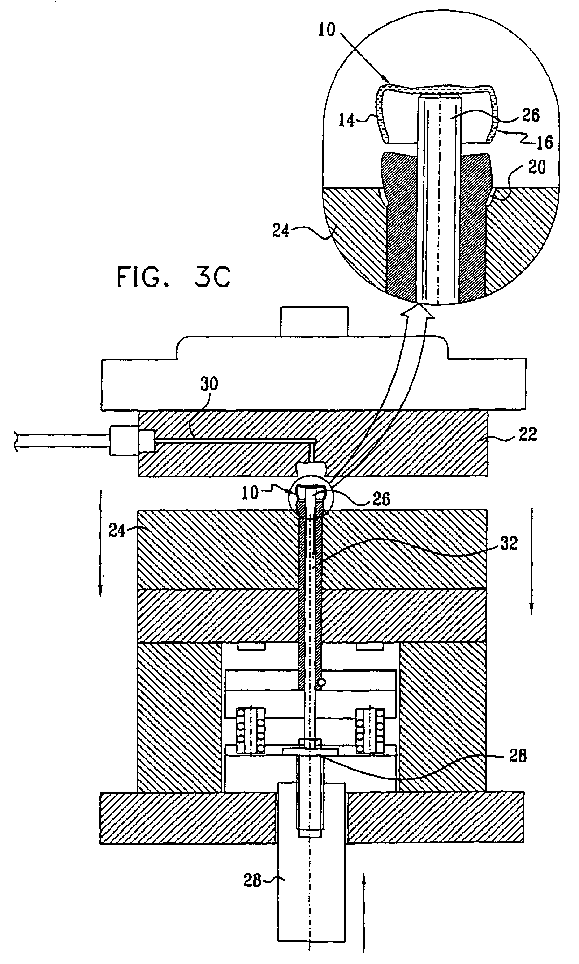

Reference is now made to Figs. 3A, 3B and 3C, which are each simplified pictorial illustrations of an apparatus for manufacturing a dental crown from acetal homopolymer resin in three operative orientations, the apparatus not being part of the invention

As seen in Figs. 3A, 3B and 3C, the crown 10 is molded in a mold cavity 20 which is defined by a top mold element 22, a bottom mold element 24 and an ejector 26. The ejector 26 forms part of an internal mold element 32.

Fig. 3A shows the stage of molding when the top mold element 22 lies in tight engagement with the bottom mold element 24 and the ejector 26. The dental crown 10, which is fabricated on the ejector 26, is formed by the injection of acetal homopolymer resin material from a source of acetal homopolymer resin (not shown) into the mold cavity 20, via a channel 30 cut in the top mold element 22.

Fig. 3B shows an initial release stage wherein the bottom mold element 24 is separated from the top mold element 22, thus permitting removal of the molded crown 10 from cavity 20.

Fig. 3C shows an ejection stage wherein ejector 26, driven by a piston 28 moves upwardly relative to bottom mold element 24 and pushes crown 10 out of cavity 20. Due to the resilience of the depending side surfaces 14, the action of the ejector 26 is able to disengage the internal mold element 32 from the crown 10 notwithstanding the presence of undercut 16:

## Claims

1. An injection molded dental crown (10) configured as a single-layer continuous structure made of a piece of acetal homopolymer resin, the dental crown being formed by a top surface (12) and flexible depending side surfaces (14) extending therefrom, with at least one of the inwardly directed side surfaces (14) defining an undercut (16) the dental crown (10) being thereby resilient and dimensionally stable to return to its original shape upon being applied to and removed from a patient's dentition and being useful for treatment of primary teeth and permanent molars in pediatric dentistry.

2. A dental crown according to claim 1, wherein said acetal homopolymer resin comprises polioxymethylene thermoplastic homopolymer.

## Patentansprüche

1. Spritzgegossene Dentalkrone (10), die als aus einem Stück Acetal-Homopolymerharz bestehende einschichtige kontinuierliche Struktur ausgebildet ist, wobei die Dentalkrone von einer oberen Oberfläche (12) und sich davor erstreckenden flexiblen abhängigen Seitenoberflächen zu gebildet wird, wobei wenigstens eine der einwärts gerichteten Seitenoberflächen zu einen Hinterschnitt (16) definiert, wodurch die Dentalkrone (10) nachgiebig und dimensional stabil wird, um beim Anwender auf und Entfernen von dem Gebiß eines Patienten in ihre Originalform zurückzukehren und nützlich für dir Behandlung der ersten Zähne und der bleibenden Backenzähne in der Kinderzahnheilkunde zu sein.

2. Dentalkrone nach Anspruch 1, wobei das Acetal-Homopolymerharz ein thermoplastisches Polyoxymethylen-Homopolymer umfaßt.

## Revendications

1. Couronne dentaire (10) modulée par injection configurée comme une structure continue monocouche, faite en un morceau de résine homopolymère d'acétal, la couronne dentaire étant formée par une surface supérieure (12) et des surfaces latérales (14) dépendantes flexibles s'étendant à partir de celle-ci, avec au moins une des surfaces latérale (14) dirigées vers l'intérieur définissant une contre-dépouille (16), la couronne dentaire (10) étant ainsi élastique et dimensionnellement stable pour reprendre sa forme d'origine lorsqu'elle est appliquée sur et retirée de la denture d'un patient et étant utile pour le traitement des dents de lait et des molaires permanentes en pédodontie.

2. Couronne dentaire selon la revendication 1, dans laquelle ladite résine homopolymère d'acétal comprend l'homopalymére thermoplastique du polyoxyméthyléne.
